# FASCICULE DE BREVET EUROPEEN

(11) **EP 4 093 485 B1**
(45) Date de publication et mention de la délivrance du brevet: **24.04.2024**
(21) Numéro de dépôt: 21701273.1
(22) Date de dépôt: 19.01.2021
(51) Int. Cl.: A61N 1/04, A61H 39/00, A61N 1/36

(54) **DISPOSITIF PORTATIF NON INVASIF DE STIMULATION NERVEUSE ÉLECTRIQUE**
NICHT-INVASIVE AM KÖRPER TRAGBARE VORRICHTUNG ZUR ELEKTRISCHEN NERVENSTIMULATION
NON-INVASIVE WEARABLE DEVICE FOR ELECTRICAL NERVE STIMULATION

(30) Priorité: 22.01.2020 FR 2000613
(43) Date de publication de la demande: 30.11.2022
(73) Titulaire: Stimuli Technology, 92100 Boulogne Billancourt (FR)
(72) Inventeur: MATTON, Yves, 92260 Fontenay aux Roses (FR); LE BLAINVAUX, Olivier, 78150 LE CHESNAY (FR); LE BLAINVAUX, Pierre, 92100 Boulogne Billancourt (FR); MARTEL, Tristan, 24230 Montcaret (FR)
(74) Mandataire: Axe PI
(86) Numéro de dépôt international: PCT/EP2021/050988
(87) Numéro de publication internationale: WO 2021/148368

(56) Documents cités:
- WO-A1-2016/183689
- WO-A1-2018/032105
- WO-A1-2020/006607
- US-A1- 2019 117 974

## Description

### Domaine technique

La présente invention concerne le domaine des dispositifs médicaux. Elle concerne plus particulièrement un dispositif portatif non invasif de stimulation nerveuse électrique ou neurostimulation électrique transcutanée (TENS), notamment pour la stimulation du nerf tibial et pour le traitement de la vessie hyperactive.

La vessie hyperactive (en anglais overactive bladder ou OAB) se traduit par un resserrement des parois internes de la vessie, quel que soit son niveau de remplissage.

La vessie hyperactive touche environ 17 % de la population mondiale et est particulièrement fréquente chez les personnes âgées.

Les signes cliniques caractéristiques d'une vessie hyperactive sont généralement :
- des envies fréquentes d'uriner (voire très fréquentes), même si la vessie n'a pas atteint sa capacité maximale de contenance (pollakiuries) pouvant notamment engendrer des réveils nocturnes pour aller uriner (nycturies) ;
- des besoins pressants de se rendre aux toilettes (urgenturies), avec l'appréhension de devoir uriner à un moment inadéquat ;
- des fuites urinaires et des incontinences lorsque la personne n'a pas la possibilité de se rendre aux toilettes ou qu'il n'y a pas de toilettes à proximité.

Parmi les facteurs de risque d'une vessie hyperactive, on peut notamment citer :
- l'infection urinaire ;
- la présence de calculs rénaux ;
- des troubles du système nerveux ; et
- une consommation accrue de boisson contenant de la caféine.

Plusieurs moyens permettent de limiter (ou de traiter) le problème d'une vessie hyperactive :
- des exercices de rééducation de la vessie, permettant de limiter les envies d'uriner;
- des médicaments spécifiques afin de réduire le nombre d'envies et de limiter les fuites ;
- une injection de toxine botulique au niveau du muscle de la vessie, permettant à la vessie de garder plus d'urine ;
- une stimulation, via un implant, du nerf sacral, situé au niveau du bassin et jouant un rôle important dans la gestion de la vessie, ou encore
- une stimulation du nerf tibial, situé au niveau de la cheville et possédant des ramifications proches du nerf sacral, permettant ainsi une stimulation indirecte de ce dernier.

Le nerf tibial est la branche terminale médiale du nerf sciatique. C'est un nerf mixte constitué de fibres nerveuses provenant des nerfs lombaires et sacrés. Le nerf tibial suit d'abord l'axe médian de la fosse poplitée puis, à la jambe, il descend médialement pour traverser successivement la région rétro malléolaire médiale et la région du tunnel tarsien. Il se divise classiquement dans le tunnel tarsien en deux branches terminales qui vont rejoindre la plante du pied : le nerf plantaire médial et le nerf plantaire latéral.

Il existe aujourd'hui un besoin de développer et d'optimiser les techniques et traitements permettant de limiter la vessie hyperactive.

Parmi les traitements existants, le traitement par neurostimulation électrique transcutanée du nerf tibial apparaît être une piste intéressante et prometteuse.

### Technique antérieure

La neurostimulation électrique transcutanée est une technique non médicamenteuse et non invasive destinée notamment à soulager la douleur à l'aide d'un courant électrique de faible tension transmis par des électrodes placées sur la peau. L'acronyme TENS, par lequel on désigne souvent cette thérapeutique, vient de son appellation anglaise « Transcutaneous Electrical Nerve Stimulation ». L'appareil qui génère le courant voulu et auquel sont reliées les électrodes est appelé « neurostimulateur».

Les électrodes, généralement au nombre de deux ou de quatre, sont fixées sur la peau à l'aide d'un ruban adhésif. Elles sont placées près de la région douloureuse ou, suivant les cas, le long du trajet d'un nerf spécifique, le nerf tibial généralement, ou à d'autres emplacements stratégiques. La personne est invitée à ajuster le neurostimulateur de manière à trouver l'intensité, la fréquence et la durée des pulsations qui lui procurent le meilleur soulagement tout en causant le minimum d'inconfort.

Les réglages, ainsi que la durée et la fréquence des séances de traitement, peuvent varier considérablement d'une personne à l'autre. Dans certains cas, l'effet analgésique ou le soulagement se font sentir immédiatement, tandis que dans d'autres, il faudra 30 minutes ou une heure de traitement avant de pouvoir être soulagé. Pour certaines personnes, l'effet analgésique ou le soulagement disparaissent aussitôt qu'on interrompt le traitement ; pour d'autres, le soulagement peut se prolonger durant des heures, voire des jours.

Comme indiqué ci-dessus, les neurostimulateurs sont notamment indiqués pour la prévention ou le traitement de la vessie hyperactive (en anglais overactive bladder ou OAB), mais peuvent également être indiqués pour la prévention ou le traitement des troubles anorectaux et des douleurs périnéales.

Les troubles anorectaux désignent les désordres du transit qui ont lieu au niveau du côlon, tels que la constipation et l'incontinence fécale. Ces troubles toucheraient notamment 39 à 66 % des personnes atteintes de sclérose en plaques et auraient des répercussions sur les activités sociales chez 1 personne sur 6. Incontinence fécale et constipation peuvent être rencontrées séparément mais le plus souvent elles se manifestent en même temps. Elles sont, de plus, fréquemment associées à des troubles urinaires.

Les douleurs périnéales sont particulièrement difficiles à traiter, nécessitant fréquemment une approche multidisciplinaire. Parmi elles, sont de mieux en mieux identifiées les névralgies pudendales, ilioinguinales, ilio-hypogastriques, génito-fémorales, les douleurs à composante ostéoligamentaire, le syndrome myofascial ainsi que les douleurs d'origine musculaire et veineuse.

Il a été montré que la neuromodulation du nerf tibial par un neurostimulateur externe permettait de délivrer des impulsions électriques indolores et de faible intensité sur le trajet dudit nerf, par l'intermédiaire d'électrodes collées sur la peau. La neuromodulation du nerf tibial postérieur consiste à stimuler les afférences sensitives de ce nerf, qui appartiennent au même territoire métamérique que les racines sacrées qui vont commander le système vésico-sphinctérien, le système anorectal et qui véhiculent les voies de la douleur du périnée.

Il existe aujourd'hui différents neurostimulateurs ou appareils d'électrostimulation périnéale. On peut notamment citer l'UROSTIM 2^{™} commercialisé par SCHWA-MEDICO^{™}, pour traiter l'incontinence et le renforcement du plancher pelvien. Il s'agit d'un appareil avec deux voies qui propose une électrothérapie périnéale. Celle-ci peut s'effectuer de différentes manières :
- stimulation périnéale avec une sonde pour raffermir les muscles du périnée et soulager tous les types d'incontinence (effort, impériosité ou urgence, et mixte);
- stimulation du NTP (Nerf Tibial Postérieur) par électrodes.

L'UROSTIM 2^{™} est un appareil d'électrostimulation rechargeable qui fonctionne sur batterie.

Le seuil de stimulation est déterminé par la perception sensitive de la stimulation par le patient. Les paramètres de stimulation habituellement utilisés pour les troubles urinaires et anorectaux sont :
- fréquence 10Hz, intensité 10 à 45 mA, intervalles 200 microsecondes.

La stimulation est réalisée à domicile, par le patient lui-même, au rythme de 20 à 30 minutes par jour durant au minimum trois mois. Le résultat clinique est évalué à l'issue de cette période. En cas d'amélioration significative la stimulation peut être poursuivie.

La fréquence peut alors être adaptée au cas par cas en fonction du résultat (une séance deux à trois fois par semaine).

Pour ce type de dispositif, il est recommandé de réaliser la séance de stimulation au repos, en position assise ou allongée.

D'autres neurostimulateurs ont été proposés. On peut citer notamment le neurostimulateur décrit dans le brevet US 9,254,382 qui est un dispositif portatif délivrant des stimuli au niveau de la cheville par des électrodes particulières. Un tel dispositif permet a priori le contrôle de l'activité physique de la vessie. Le dispositif décrit comprend un boîtier, un circuit générateur d'impulsions permettant de générer un stimulus d'électro-acupuncture et une sangle pour la fixation du boîtier directement à la cheville. Le dispositif comprend également une paire d'électrodes en forme de D. Le document WO2018032105 décrit par exemple un appareil doté des capacités similaires à celui de l'invention.

### Résumé de l'invention

La présente invention concerne le domaine des dispositif médicaux. Elle concerne plus particulièrement un dispositif portatif non invasif de stimulation nerveuse électrique ou neurostimulation électrique transcutanée (TENS), notamment pour la stimulation du nerf tibial et pour le traitement de la vessie hyperactive.

### Problème technique

Considérant ce qui précède, un problème que se propose de résoudre la présente invention consiste à développer un nouveau dispositif de neurostimulation électrique transcutanée qui présente l'avantage d'être simple d'utilisation, qui soit facilement manipulable, compatible avec les contraintes de la vie courante et qui permette une stimulation quotidienne ou quasi-quotidienne du nerf tibial sur des durées longues (plusieurs heures) sans représenter un handicap pour le patient. Plus particulièrement, le dispositif doit permettre de limiter la gêne liée à son utilisation. De façon additionnelle, le dispositif devrait pouvoir être utilisé lorsque l'utilisateur est en activité et non pas seulement au repos, allongé ou assis.

### Solution technique

La solution à ce problème posé a pour premier objet un dispositif portatif non invasif de stimulation nerveuse électrique 1, comprenant :
- un module monobloc électrique 2 dont la surface inférieure 22 est adaptée à être appliquée à la surface extérieure 9 d'un corps humain, ledit module comprenant un boîtier 3 intégrant un circuit générateur d'impulsions 4 connecté à au moins deux électrodes 51, 52, préférentiellement des électrodes en silicone-graphite, lesdites électrodes étant séparées par un espace 50 d'au moins 50 mm pour délivrer une impulsion électrique au nerf positionné dans le corps ; et
- un moyen de fixation 6 dudit module 2 au corps humain ;
caractérisé en ce que le moyen de fixation 6 est un système d'attache unique positionné sur la partie supérieure d'une cheville, au-dessus de la malléole.

L'invention a également pour objet l'utilisation d'un dispositif selon l'invention, pour la stimulation nerveuse, préférentiellement du nerf tibial pour le traitement de la vessie hyperactive.

L'invention a aussi pour objet un dispositif selon l'invention, pour son utilisation dans la prévention ou le traitement de la vessie hyperactive par stimulation du système nerveux, préférentiellement le nerf tibial, plus préférentiellement le nerf tibial postérieur.

L'invention a aussi pour objet une méthode de prévention ou de traitement de la vessie hyperactive par stimulation du système nerveux par un dispositif portatif non invasif de stimulation nerveuse électrique selon l'invention, préférentiellement pour la stimulation du nerf tibial, plus préférentiellement du nerf tibial postérieur.

L'invention a aussi pour objet un procédé de contrôle de la vessie hyperactive par stimulation du système nerveux par un dispositif portatif non invasif de stimulation nerveuse électrique selon l'invention, préférentiellement pour la stimulation du nerf tibial, plus préférentiellement du nerf tibial postérieur.

L'invention a aussi pour objet un kit adapté à la mise en oeuvre d'un procédé de contrôle selon l'invention, comprenant un dispositif tel que décrit ci-après, et préférentiellement une notice d'utilisation pour l'administration de ladite stimulation nerveuse électrique.

### Avantages apportés

Le Demandeur a notamment pu développer un dispositif portatif non invasif de stimulation nerveuse électrique qui présente un encombrement réduit, des qualités de légèreté et une facilité d'utilisation. De plus, le dispositif dispose avantageusement de moyens permettant de faciliter son utilisation, y compris lorsque l'utilisateur est chaussé. Le Demandeur a pu développer un dispositif dont le design permet de s'adapter au port de chaussures basses, sans pour autant modifier ou limiter la neurostimulation. Un tel dispositif est donc plus facilement et fréquemment porté sur des durées longues (plusieurs heures) par son utilisateur.

### Brève description des dessins

L'invention et les avantages qui en découlent seront mieux compris à la lecture de la description et des modes de réalisation non limitatifs qui suivent, illustrés au regard des dessins annexés dans lesquels :
[fig. 1] représente une vue de profil d'un pied d'utilisateur équipé d'un dispositif selon l'invention.
[fig. 2] représente une vue de face d'un pied d'utilisateur équipé d'un dispositif selon l'invention.
[fig. 3] représente un schéma d'un circuit générateur d'impulsions d'un dispositif selon l'invention.

### Description des modes de réalisation

Dans cette description, à moins qu'il ne soit spécifié autrement, il est entendu que, lorsqu'un intervalle est donné, il inclut les bornes supérieure et inférieure dudit intervalle.

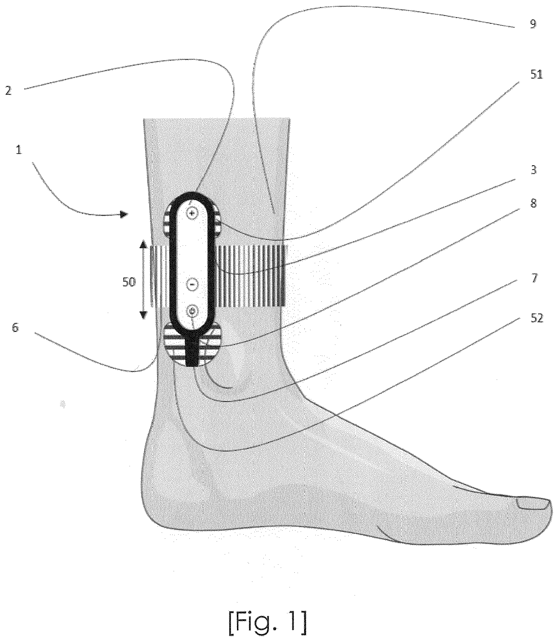

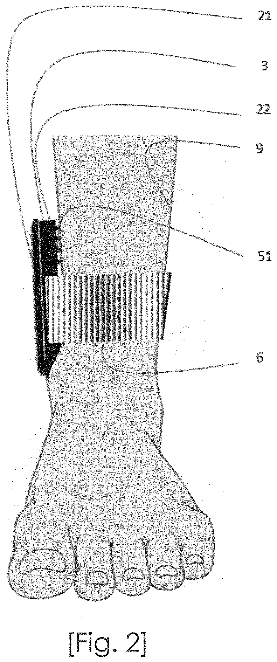

Comme il ressort des figures 1 et 2, l'invention a pour objet un dispositif portatif non invasif, c'est-à-dire qui ne requiert aucune incision ou effraction sur la peau. Le dispositif est un dispositif de stimulation nerveuse électrique 1, également appelé neurostimulateur. Il s'agit d'un appareil qui génère un courant voulu aux électrodes 51 et 52. Le dispositif est portatif en ce qu'il peut être transporté facilement, et qu'il est sans-fil, donc non relié à une prise et sans fil apparent qui risquerait par exemple d'être sectionné, coupé ou dénudé lors de son utilisation. Ceci est avantageux et garantie une sécurité d'utilisation.

Le dispositif 1 comprend un module monobloc électrique 2 avec une face supérieure 21 qui comprend un ou plusieurs boutons de commande 8. De préférence, le dispositif 1 comprend au moins un bouton de commande 8 sur sa surface supérieure 21 permettant d'allumer et d'éteindre le dispositif. Le même bouton ou un bouton additionnel permet de régler l'intensité et/ou la durée du traitement. Ceci rend le dispositif particulièrement ergonomique.

La surface inférieure 22 du module monobloc 2 est quant à elle adaptée à être appliquée à la surface extérieure 9 d'un corps humain. De préférence, la surface inférieure 22 est appliquée sur la peau, plus préférentiellement au niveau du nerf tibial, c'est-à-dire sur la partie supérieure de la cheville, au-dessus de la malléole comme illustré aux figures 1 et 2.

Le dispositif 1 selon l'invention comprend un boîtier 3 qui intègre un circuit générateur d'impulsion 4 connecté à au moins deux électrodes 51,52.

De préférence, les électrodes sont solidaires du produit ou du boitier 3.

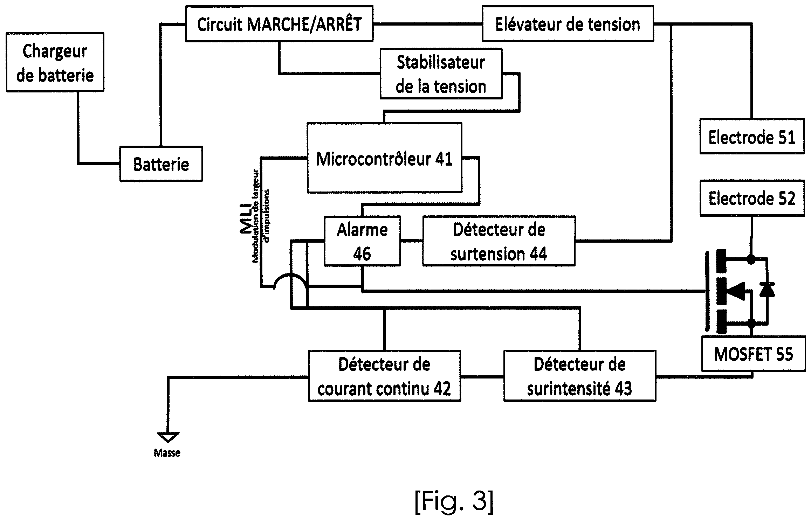

Comme illustré à la figure 3, le circuit est avantageusement constitué de différents modules permettant de délivrer un courant aux électrodes.

Le circuit 4 intègre préférentiellement plusieurs des éléments suivants :
- un chargeur de batterie qui est préférentiellement un chargeur micro-USB, USB ou USB-C ;
- une batterie qui est par exemple une batterie rechargeable de type Nickel Cadmium, Nickel métal-hydrure, lithium-ion, lithium Fer Phosphate ou Plomb possédant préférentiellement une autonomie en fonctionnement nominale de 24h ou plus ou de 20h ou plus ;
- un circuit marche/arrêt ;
- un élévateur de tension ;
- un stabilisateur de tension ;
- un microcontrôleur 41 qui permet de contrôler les paramètres de stimulation ;
- une alarme 46;
- un détecteur de surtension 44, à fonction matérielle uniquement ;
- un détecteur de courant continu 42 relié à la masse, à fonction matérielle uniquement ;
- un détecteur de surintensité 43, à fonction matérielle uniquement ;
- un transistor à effet de champ à grille isolée (55) (MOSFET pour *Métal Oxide Semiconductor Field Effect Transistor*) destiné à fermer le circuit pour déclencher la stimulation ;
et est relié aux deux électrodes 51,52.

De préférence, le circuit générateur d'impulsions 4 du dispositif selon l'invention comprend :
- une batterie qui est par exemple une batterie rechargeable de type Nickel Cadmium, Nickel métal-hydrure, lithium-ion, lithium Fer Phosphate ou Plomb possédant préférentiellement une autonomie en fonctionnement nominale de 24h ou plus ou de 20h ou plus;
- un microcontrôleur (41) ;
- un détecteur de courant continu (42) ;
- un détecteur de surintensité (43) ;
- un détecteur de surtension (44) ;
- un transistor à effet de champ à grille isolée (55) (MOSFET pour *Métal Oxide Semiconductor Field Effect Transistor*) ; et éventuellement
- une alarme (46) ;
pour arrêter la stimulation en cas de dysfonctionnement.

De façon avantageuse, le Demandeur a intégré un microcontrôleur 41 au circuit générateur d'impulsions 4 du dispositif selon l'invention afin d'augmenter la sécurité du dispositif.

Par ailleurs, lorsqu'une tension seuil, une intensité seuil ou un courant continu sont détectés, une sécurité matérielle (hardware) permet avantageusement de couper la stimulation.

Comme illustré aux figures 1 à 3, le circuit générateur d'impulsions 4 est connecté à au moins deux électrodes 51, 52. Les électrodes sont des éléments capables de conduire le courant électrique, et sont utilisées pour stimuler le système nerveux, notamment le nerf tibial.

De préférence, les électrodes sont non-adhésives. Elles évitent ainsi un toucher ou aspect collant non agréable pour l'utilisateur. De telles électrodes non-adhésives permettent ainsi une utilisation plus régulière, meilleure et plus constante par l'utilisateur.

Les électrodes du dispositif selon l'invention sont préférentiellement en silicone graphite. Elles sont espacées d'au moins 50 mm et délivrent une impulsion électrique au nerf positionné dans le corps, préférentiellement le nerf tibial. De préférence les électrodes 51, 52 sont espacées d'au moins 70 mm, plus préférentiellement au moins 80 mm, plus préférentiellement encore au moins 90 mm. De façon particulièrement avantageuse, la distance entre les deux électrodes est comprise entre 50 mm et 150 mm, préférentiellement entre 75 mm et 125 mm, plus préférentiellement encore entre 80 mm et 100 mm.

Le Demandeur a notamment pu mettre en évidence qu'il était déconseillé d'avoir une distance entre les électrodes 51, 52 inférieure à 50 mm pour toucher efficacement le nerf tibial.

Le module monobloc comprend avantageusement, sur sa partie basse, un téton 7 permettant de relier le boîtier 3 du module à une électrode basse 52, permettant ainsi le passage libre de la malléole sous ledit dispositif 1.

La présence de ce téton permet de limiter l'encombrement général du dispositif. Par ailleurs, cette diminution de l'encombrement du dispositif permet à l'utilisateur de se chausser ou de porter des chaussures basses, tout en gardant le dispositif au-dessus de sa cheville et peut être porté sur des durées longues (plusieurs heures). Ainsi, le dispositif s'adapte notamment à toutes les largeurs de chevilles sans risque de gêner l'utilisateur, par exemple par contact entre une partie rigide et la malléole interne.

Ainsi, l'utilisateur n'est pas obligé d'être déchaussé lorsqu'il utilise le dispositif selon l'invention qui est un neurostimulateur.

Le dispositif selon l'invention comprend en outre un moyen de fixation 6 du module 2 au corps humain qui est composé d'un matériau choisi parmi le polyester, le polyamide, le caoutchouc, pris seuls ou en combinaison. Comme illustré aux figures 1 et 2, le moyen de fixation 6 est un système d'attache unique positionné sur la partie supérieure d'une cheville, au-dessus de la malléole.

De préférence, le moyen de fixation 6 est une sangle unique ou une bande unique adaptée pour que les électrodes 51, 52 du module soient directement en contact avec la peau du corps humain 9 lorsque le dispositif est positionné sur le corps.

Alternativement, le moyen de fixation 6 peut également se présenter sous la forme d'un brassard.

Le dispositif comprend avantageusement au moins un bouton de commande 8 sur sa surface supérieure 21 permettant de régler l'alimentation, l'intensité et/ou la durée du traitement.

L'intensité du traitement varie généralement entre 0 et 150 mA, de préférence, l'intensité est comprise entre 0 et 50 mA, plus préférentiellement elle est comprise entre 10 et 45 mA.

La forme préférée de stimulation ou de signal est une forme de créneau. La fréquence de stimulation ou de pulse est comprise entre 5 à 30 Hz, préférentiellement entre 10 et 20 Hz.

La largeur du créneau ou durée de pulse est généralement comprise entre 50 et 300 microsecondes, préférentiellement entre 150 à 250 microsecondes, plus préférentiellement entre 150 et 200 microsecondes.

La durée de pulse peut avantageusement être modulée par modulation à largeur d'impulsion (MLI).

Enfin, la durée de stimulation par jour est comprise entre 1 minute et 24 heures, préférentiellement entre 10 minutes et 22 heures, plus préférentiellement entre 20 minutes et 20 heures.

Le dispositif selon l'invention comprend avantageusement un module monobloc 2 qui est composé d'un matériau choisi parmi le caoutchouc silicone, l'acrylonitrile butadiène styrène, copolymère à blocs de styrène-éthylène-éthylène-propylène-styrène, le polycarbonate, pris seuls ou en combinaison ; et des électrodes 51, 52 en silicone-graphite.

Le dispositif selon l'invention intègre avantageusement un module d'échange de données numériques sans fil, dont le réseau sans fil met en oeuvre une norme du type 802.11, Bluetooth ou DECT.

Le module d'échange de données numériques sans fil permet notamment l'activation à distance et à la demande.

De façon alternative, le dispositif selon l'invention peut avantageusement être un objet connecté à internet et entrer dans l'appellation internet des objets dit « IdO » ou « IoT » (de l'anglais Internet of things). L'appellation désigne un nombre croissant d'objets connectés à Internet permettant ainsi une communication entre nos biens dits physiques et leurs existences numériques. Le réseau doit permettre d'envoyer des très petits messages sur des longues portées sans nécessairement passer par des systèmes de réseaux mobile et en consommant peu d'énergie.

Le volume d'encombrement du module monobloc électrique 2 du dispositif selon l'invention est avantageusement inférieur à 90 cm³, préférentiellement inférieur à 75 cm³, plus préférentiellement encore inférieur à 70 cm³.

Plus particulièrement, le dispositif selon l'invention présente avantageusement les caractéristiques suivantes :
- la hauteur (H) du module monobloc électrique 2 est inférieure à 150 mm, préférentiellement inférieure à 100 mm ;
- la largeur (L) du module monobloc électrique 2 est inférieure à 40 mm, préférentiellement inférieure à 30 mm ;
- la profondeur (P) du module monobloc électrique 2 est inférieure à 15 mm, préférentiellement inférieure à 10 mm.

Le dispositif selon l'invention est un neurostimulateur qui permet donc une neurostimulation électrique transcutanée, plus communément appelée "TENS" (de l'anglais Transcutaneous Electrical Nerve Stimulation). L'utilisation du dispositif est donc une technique non médicamenteuse et non-invasive permettant soit de soulager une douleur, soit de prévenir ou traiter certains désordres tels que la vessie hyperactive (en anglais overactive bladder ou OAB), mais également les troubles anorectaux et les douleurs périnéales.

Ainsi, le dispositif peut notamment être utilisé :
- d'une part pour un inhiber le signal douloureux : le dispositif selon l'invention génère des impulsions électriques qui activent des fibres nerveuses de plus gros calibre, et plus rapides que celles utilisées pour véhiculer la douleur. Un message est transmis au cerveau par l'intermédiaire de la moelle épinière, et masque ainsi le signal douloureux pendant la durée de la séance de stimulation ;
- d'autre part pour une stimulation endorphinique, qui favorise l'augmentation de la production d'endorphines. Cette augmentation entraîne un effet antalgique général. La TENS en mode endorphinique se caractérise par une sensation de petits battements.

Les programmes TENS proposent l'un ou l'autre de ces mécanismes d'action ou une combinaison des deux. Le type de programme et le positionnement des électrodes sont déterminés lors d'une séance de test avec un professionnel de santé. Le choix s'effectue selon le ressenti de chaque patient.

Le dispositif selon l'invention est un dispositif miniaturisé et léger, son design lui permet d'être fixé sur de nombreuses parties du corps par l'intermédiaire de la sangle unique qui peut faire office de sangle ou de brassard. Le dispositif est compact et peut facilement être dissimulé sous des vêtements, de préférence des vêtements amples (pantalon, chemise, etc.). Il permet de bouger et de vivre normalement sans se préoccuper de l'encombrement du dispositif.

Il s'agit d'un dispositif médical personnel, utilisable à tout moment de la journée sur des durées variables, en fonction des épisodes.

De préférence, les électrodes du dispositif sont positionnées au-dessus de la malléole interne et délivrent une impulsion électrique au nerf tibial postérieur.

La présente invention va maintenant être illustrée au moyen des exemples suivants.

### Exemples

### Exemple 1 : Comparatif et test d'utilisation d'un dispositif selon l'invention (version initiale ou version améliorée TENSI+)

- Intensité : 0 - 50 mA
- Forme de stimulation : créneau
- Largeur du créneau : 150-200 microsecondes
- Fréquence de stimulation : 10-20 Hz
- Durée de stimulation par jour : 20 min à 20 heures

Le circuit du dispositif comprend les éléments suivants :
- un chargeur de batterie qui est préférentiellement un chargeur micro-USB, USB ou USB-C ;
- une batterie qui est par exemple une batterie rechargeable de type Nickel Cadmium, Nickel métal-hydrure, lithium-ion, lithium Fer Phosphate ou Plomb possédant une autonomie en fonctionnement nominale de 24h préférentiellement supérieure à 20h ;
- un circuit marche/arrêt ;
- un élévateur de tension ;
- un stabilisateur de tension ;
- un microcontrôleur 41 qui permet de contrôler les paramètres de stimulation ;
- une alarme 46;
- un détecteur de surtension 44, à fonction matérielle uniquement ;
- un détecteur de courant continu 42 relié à la masse, à fonction matérielle uniquement ;
- un détecteur de surintensité 43, à fonction matérielle uniquement ;
- un transistor à effet de champ à grille isolée (55) (MOSFET pour Metal Oxide Semiconductor Field Effect Transistor) destiné à fermer le circuit pour déclencher la stimulation ;
et est relié à deux électrodes 51,52.

Le modèle TENSI+ comprend en outre un téton 7 permettant de relier le boîtier 3 du module à une électrode basse 52, permettant ainsi le passage libre de la malléole sous le dispositif selon l'invention.

Tab. 1. Les résultats du comparatif sont repris dans le tableau 1 ci-dessous.

**[Tableau 1]**

| | Design évalué | | Urostim 2.0 | | Design initial | | Design TENSI+ |
|---|---|---|---|---|---|---|---|
| | Le fonctionnement du produit est simple et intuitif | | 3,1 | | 3,2 | | 4,1 |
| | Le produit est facile à installer | | 2,6 | | 2,7 | | 4,0 |
| | Le produit est confortable à porter | | 1,9 | | 2,1 | | 4,0 |
| | Je peux porter le dispositif au quotidien à la maison (20-30 min) | | 2,7 | | 3,6 | | 4,1 |
| | Je peux porter des chaussures avec le dispositif | | 1,7 | | 2,6 | | 4,0 |
| | Le dispositif n'est pas stigmatisant | | 1,6 | | 3,6 | | 3,8 |
| Moyenne | | 2,23 | | 2,94 | | 3,98 | |

| | | | | | | | |
|---|---|---|---|---|---|---|---|
| Note de 1 (pas du tout) à 5 (tout à fait) pour chaque question | | | | | | | |

### Conclusion :

Trois dispositifs ont été évalués par un groupe de 20 participants afin de noter sur une échelle allant de 1 à 5 (1 = pas du tout et 5 = tout à fait) la facilité d'utilisation et l'ergonomie de ces trois produits.

Ces trois dispositifs sont les suivants :
- Urostim 2.0 : dispositif déjà commercialisé ;
- Design initial : première version du dispositif selon l'invention ;
- TENSI+ : dernière version améliorée du dispositif selon l'invention comprenant un téton permettant un passage facilité de la malléole.

Une série de questions a été posée aux utilisateurs ayant testés ces dispositifs pendant plusieurs jours.

Les dispositifs selon l'invention apparaissent clairement présenter des avantages par rapport au dispositif de l'art antérieur.

Plus particulièrement, le dispositif TENSI+ obtient la meilleure moyenne avec la plus haute note pour chacune des questions posées aux utilisateurs.

Les dispositifs selon l'invention de manière générale, et plus particulièrement le dispositif TENSI+, apparaissent être les plus pratiques et présenter une facilité d'utilisation supérieure au dispositif de l'art antérieur grâce à un encombrement réduit s'adaptant au port de chaussures, sans pour autant modifier ou limiter la neurostimulation. De plus, les dispositifs selon l'invention peuvent être utilisés quotidiennement sur des durées longues (plusieurs heures) par l'utilisateur au repos ou pendant ses activités tout en restant confortables à porter.

## Revendications

1. Dispositif portatif non invasif de stimulation nerveuse électrique (1), comprenant :
- un module monobloc électrique (2) dont la surface inférieure (22) est adaptée à être appliquée à la surface extérieure (9) d'un corps humain, ledit module comprenant un boîtier (3) intégrant un circuit générateur d'impulsions (4) connecté à au moins deux électrodes (51, 52), lesdites électrodes étant séparées par un espace (50) d'au moins 50 mm pour délivrer une impulsion électrique audit nerf positionné dans le corps ; et
- un moyen de fixation (6) dudit module (2) au corps humain qui est un système d'attache unique positionné sur la partie supérieure d'une cheville, au-dessus de la malléole **caractérisé en ce que** le module monobloc comprend, sur sa partie basse, un téton (7) permettant de relier le boîtier (3) du module à une électrode basse (52), permettant ainsi le passage libre de la malléole sous ledit dispositif (1).

2. Dispositif selon la revendication 1, **caractérisé en ce que** les électrodes (51, 52) sont des électrodes en silicone-graphite

3. Dispositif selon l'une des revendications 1 ou 2, **caractérisé en ce que** le moyen de fixation (6) est :
- une sangle unique ou une bande unique, composée d'un matériau choisi parmi le polyester, le polyamide, le caoutchouc, pris seuls ou en combinaison ;
- adapté pour que les électrodes (51, 52) du module soient directement en contact avec la peau du corps humain (9) lorsque le dispositif est positionné sur le corps.

4. Dispositif selon l'une des revendications précédentes, **caractérisé en ce qu'**il comprend au moins un bouton de commande (8) sur sa surface supérieure (21).

5. Dispositif selon l'une des revendications précédentes, **caractérisé en ce que** le module monobloc (2) est composé d'un matériau choisi parmi le caoutchouc silicone, l'acrylonitrile butadiène styrène, copolymère à blocs de styrène-éthylène-éthylène-propylène-styrène, le polycarbonate, pris seuls ou en combinaison ; et des électrodes 51, 52 en silicone-graphite.

6. Dispositif selon l'une des revendications précédentes, **caractérisé en ce que** le circuit générateur d'impulsions comprend :
- une batterie qui est par exemple une batterie rechargeable de type Nickel Cadmium, Nickel métal-hydrure, lithium-ion, lithium Fer Phosphate ou Plomb possédant préférentiellement une autonomie en fonctionnement nominale de 24h ou plus ;
- un microcontrôleur (41) ;
- un détecteur de courant continu (42) ;
- un détecteur de surintensité (43) ;
- un détecteur de surtension (44) ;
- un transistor à effet de champ à grille isolée (55) (MOSFET pour Metal Oxide Semiconductor Field Effect Transistor) ; et éventuellement
- une alarme (46) ;
pour arrêter la stimulation en cas de dysfonctionnement.

7. Dispositif selon l'une des revendications précédentes, **caractérisé en ce que** le volume d'encombrement du module monobloc électrique (2) est inférieur à 90 cm3, préférentiellement inférieur à 70 cm3.

8. Dispositif selon l'une des revendications précédentes, **caractérisé en ce que** :
- la hauteur (H) du module monobloc électrique (2) est inférieure à 150 mm, préférentiellement inférieure à 100 mm ;
- la largeur (L) du module monobloc électrique (2) est inférieure à 40 mm, préférentiellement inférieure à 30 mm ;
- la profondeur (P) du module monobloc électrique (2) est inférieure à 15 mm, préférentiellement inférieure à 10 mm.

9. Dispositif selon l'une des revendications précédentes, pour son utilisation dans la prévention ou le traitement de la vessie hyperactive par stimulation du système nerveux.

10. Dispositif pour son utilisation selon la revendication 9, **caractérisé en ce qu'**il stimule le nerf tibial, préférentiellement le nerf tibial postérieur.

## Patentansprüche

1. Nichtinvasive, tragbare Vorrichtung zur elektrischen Nervenstimulation (1), umfassend:
- ein einteiliges elektrisches Modul (2), dessen untere Oberfläche (22) angepasst ist, um auf der äußeren Oberfläche (9) eines menschlichen Körpers appliziert zu werden, wobei das Modul ein Gehäuse (3) umfasst, in dem eine mit mindestens zwei Elektroden (51, 52) verbundene Impulserzeugungsschaltung (4) eingebaut ist, wobei die Elektroden zum Abgeben eines elektrischen Impulses an den in dem Körper befindlichen Nerv durch einen Zwischenraum (50) von mindestens 50 mm beabstandet sind; und
- ein Mittel (6) zur Befestigung des Moduls (2) am menschlichen Körper, das ein einziges Befestigungssystem ist, das an dem oberen Abschnitt eines Sprunggelenks oberhalb des Fußknöchels positioniert ist, **dadurch gekennzeichnet, dass** das einteilige Modul an seinem unteren Teil einen Zapfen (7) umfasst, der ermöglicht, das Gehäuse (3) des Moduls mit einer unteren Elektrode (52) zu verbinden, wodurch der freie Durchgang des Fußknöchels unter die Vorrichtung (1) ermöglicht wird.

2. Vorrichtung nach Anspruch 1, **dadurch gekennzeichnet, dass** die Elektroden (51, 52) Elektroden aus Silikon-Graphit sind

3. Vorrichtung nach einem der Ansprüche 1 oder 2, **dadurch gekennzeichnet, dass** das Mittel (6) zur Befestigung:
- ein einziger Gurt oder ein einziges Band ist, der oder das aus einem Material besteht, das aus Polyester, Polyamid, Gummi, allein genommen oder in Kombination, ausgewählt ist;
- angepasst ist, damit, wenn die Vorrichtung an dem Körper positioniert ist, die Elektroden (51, 52) des Moduls direkt in Kontakt mit der Haut des menschlichen Körpers (9) vorliegen.

4. Vorrichtung nach einem der vorstehenden Ansprüche, **dadurch gekennzeichnet, dass** sie an ihrer oberen Oberfläche (21) mindestens einen Bedienungsknopf (8) umfasst.

5. Vorrichtung nach einem der vorstehenden Ansprüche, **dadurch gekennzeichnet, dass** das einteilige Modul (2) aus einem Material, das aus Silikonkautschuk, Acrylnitril-Butadien-Styrol, Styrol-Ethylen-Ethylen-Propylen-Styrol-Blockcopolymer, Polycarbonat, allein genommen oder in Kombination, ausgewählt ist; und Elektroden 51, 52 aus Silikon-Graphit besteht.

6. Vorrichtung nach einem der vorstehenden Ansprüche, **dadurch gekennzeichnet, dass** die Impulserzeugungsschaltung umfasst:
- eine Batterie, die zum Beispiel eine wiederaufladbare Batterie vom Nickel-Cadmium-, Nickel-Metallhydrid-, Lithium-Ionen-, Lithium-Eisen-Phosphat- oder Blei-Typ ist und vorzugsweise eine Nennbetriebsdauer von 24 Stunden oder mehr besitzt;
- einen Mikrocontroller (41);
- einen Gleichstromdetektor (42);
- einen Überstromdetektor (43);
- einen Überspannungsdetektor (44);
- einen Feldeffekttransistor mit isoliertem Gate (55) (MOSFET - Metal Oxide Semiconductor Field Effect Transistor); und optional
- ein Alarmsystem (46);
zum Beenden der Stimulation im Fall einer Fehlfunktion.

7. Vorrichtung nach einem der vorstehenden Ansprüche, **dadurch gekennzeichnet, dass** das Raumbedarfvolumen des elektrischen einteiligen Moduls (2) weniger als 90 cm3, vorzugsweise weniger als 70 cm3, beträgt.

8. Vorrichtung nach einem der vorstehenden Ansprüche, **dadurch gekennzeichnet, dass**:
- die Höhe (H) des einteiligen elektrischen Moduls (2) weniger als 150 mm, vorzugsweise weniger als 100 mm, beträgt;
- die Breite (L) des einteiligen elektrischen Moduls (2) weniger als 40 mm, vorzugsweise weniger als 30 mm, beträgt;
- die Tiefe (P) des einteiligen elektrischen Moduls (2) weniger als 15 mm, vorzugsweise weniger als 10 mm, beträgt.

9. Vorrichtung nach einem der vorstehenden Ansprüche zur Verwendung bei der Vorbeugung oder der Behandlung einer überaktiven Harnblase durch Stimulation des Nervensystems.

10. Vorrichtung zur Verwendung nach Anspruch 9, **dadurch gekennzeichnet, dass** sie den Nervus tibialis, vorzugsweise den Nervus tibialis posterior, stimuliert.

## Claims

1. Portable non-invasive electrical nerve stimulation device (1), comprising:
- an electrical one-piece module (2) of which the lower surface (22) is suitable for being applied to the exterior surface (9) of a human body, said module comprising a housing (3) incorporating a pulse generator circuit (4) connected to at least two electrodes (51, 52), said electrodes being separated by a space (50) of at least 50 mm to output an electrical pulse to said nerve positioned in the body; and
- means (6) for fastening said module (2) to the human body, which is a single fastening system positioned on the upper portion of an ankle, above the malleolus, **characterized in that** the one-piece module comprises, on its lower portion, a stud (7) making it possible to connect the housing (3) of the module to a lower electrode (52), thus making possible the free passage of the malleolus under said device (1).

2. Device according to claim 1, **characterized in that** the electrodes (51, 52) are silicone-graphite electrodes.

3. Device according to one of claims 1 or 2, **characterized in that** the fastening means (6) is:
- a single strap or a single strip, composed of a material chosen from polyester, polyamide, rubber, taken alone or in combination;
- suitable for the electrodes (51, 52) of the module to be directly in contact with the skin of the human body (9) when the device is positioned on the body.

4. Device according to any of the preceding claims, **characterized in that** it comprises at least one control button (8) on its upper surface (21).

5. Device according to any of the preceding claims, **characterized in that** the one-piece module (2) is composed of a material chosen from silicone rubber, acrylonitrile butadiene styrene, styrene-ethylene-ethylenepropylene-styrene blocks copolymer, polycarbonate, taken alone or in combination; and silicone-graphite electrodes 51, 52.

6. Device according to any of the preceding claims, **characterized in that** the pulse circuit generator comprises:
- a battery which is for example a nickel-cadmium, nickel-metal hydride, lithium-ion, lithium iron phosphate or lead rechargeable battery preferably having a battery life in nominal operation of 24 h or more;
- a microcontroller (41);
- a direct current detector (42);
- an overcurrent detector (43);
- a surge detector (44);
- an insulated-gate field-effect transistor (55) (MOSFET for Metal Oxide Semiconductor Field Effect Transistor); and optionally
- an alarm (46);
to stop the stimulation in case of malfunction.

7. Device according to any of the preceding claims, **characterized in that** the bulk volume of the one-piece electrical module (2) is less than 90 cm3, preferably less than 70 cm3.

8. Device according to any of the preceding claims, **characterized in that**:
- the height (H) of the one-piece electrical module (2) is less than 150 mm, preferably less than 100 mm;
- the width (L) of the one-piece electrical module (2) is less than 40 mm, preferably less than 30 mm;
- the depth (P) of the one-piece electrical module (2) is less than 15 mm, preferably less than 10 mm.

9. Device according to any of the preceding claims, for its use in the prevention or treatment of hyperactive bladder by stimulation of the nervous system.

10. Device for its use according to claim 9, **characterized in that** it stimulates the tibial nerve, preferably the posterior tibial nerve.
